# EUROPEAN PATENT SPECIFICATION

(11) **EP 2 924 753 B1**
(45) Date of publication and mention of the grant of the patent: **19.04.2017**
(21) Application number: 14161563.3
(22) Date of filing: 25.03.2014
(51) Int. Cl.: H01L 51/00, H01L 51/50, H01L 27/32, C07C 211/54, C09K 11/06

(54) **POLYCHROMATIC LIGHT EMITTING DEVICES AND VERSATILE HOLE TRANSPORTING MATRIX FOR THEM**
POLYCHROMATISCHE LICHTEMITTIERENDE VORRICHTUNGEN UND VIELSEITIGE LOCHTRANSPORTMATRIX DAFÜR
DISPOSITIFS ÉMETTANT DE LA LUMIÈRE POLYCHROMATIQUE ET MATRICE DE TRANSPORT DE TROU POLYVALENT POUR CEUX-CI

(43) Date of publication of application: 30.09.2015
(73) Proprietor: Novaled GmbH, 01307 Dresden (DE)
(72) Inventor: Zöllner, Mike, 01217 Dresden (DE); Köhler, Martin, 01307 Dresden (DE)
(74) Representative: Bittner, Thomas L.

(56) References cited:
- EP-A1- 1 696 015
- WO-A1-2007/072962
- WO-A1-2014/021572
- WO-A2-2011/139129
- DE-A1-102009 053 191
- JP-A- H05 105 647
- JP-A- 2002 241 352
- US-A1- 2012 223 633

## Description

The present invention relates to organic light-emitting devices (OLEDs), and to compound which may be used in such devices, especially in hole transporting and/or electron blocking layers thereof.

In OLEDs, the electroluminescence (EL) property of certain organic materials is used. In EL devices, suitable charge carriers are formed under application of a voltage across the device. Recombination of these charge carriers results in an excited state, which relaxes to the ground state under light emission. To increase the efficiency, the organic light-emitting diodes very often have, besides the emission layer, also charge transporting layers which are responsible for transport of negative and positive charge carriers into the emission layer. These charge transporting layers are grouped, depending on the charge carrier transported, into hole conductors and electron conductors. A quite similar set of layers is known for photovoltaic devices, such as organic solar cells. Organic semiconducting devices having several layers are produced by known methods, for example evaporation under vacuum or deposition from solution.

In other words, in case of organic light-emitting diodes, light is produced and emitted by the injection of charge carriers, electrons from one side, holes from the other, from the contacts into adjacent organic layers as a result of an externally applied voltage, subsequent formation of excitons (electron-hole pairs) and their radiative recombination in a recombination zone.

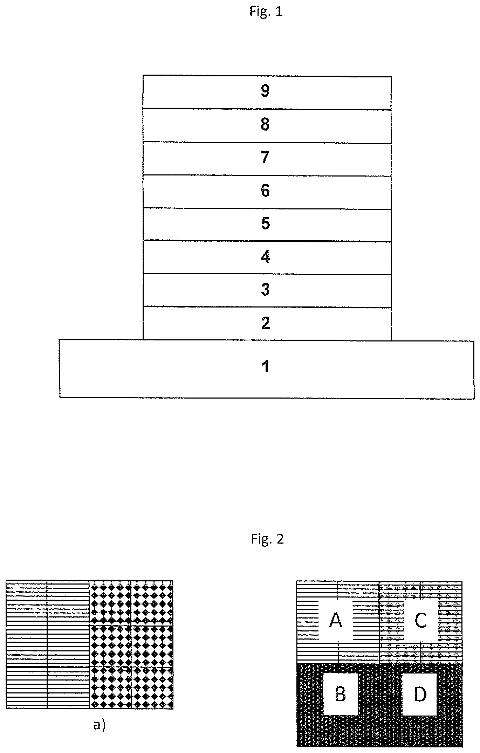

The state-of-the-art OLED structure with the positive electrode (anode) adjacent to the substrate is schematically shown in Fig. 1, wherein the numbers 1-9 denominate the following layers:
1. Transparent substrate
2. Transparent anode as bottom, hole injecting electrode
3. Hole-injecting layer
4. Hole-transporting layer (HTL)
5. Light-emitting layer (EL)
6. Electron-transporting layer (ETL)
7. Electron-injecting layer (EIL)
8. Cathode as top electrode (usually a metal with low work function, electron-injecting)
9. Encapsulation to protect from moisture, oxygen and other outside factors which may affect stability or efficiency.

While this description represents the most common case, often several layers may be omitted, or else one layer may fulfill several functions.

An important property of organic semiconducting materials is their conductivity. The conductivity of a thin layer sample can be measured by, for example, the two-point method. A voltage is applied to the thin layer and the current flowing through the layer is measured. The measured resistance or conductivity, respectively, can be calculated from the geometry of the contacts and the thickness of the layer of the sample.

In an OLED, the operational voltage (or, more exactly, the overall electrical resistance) is determined not only by resistances and thicknesses of particular layers, but also by energetic barriers for charge carrier injection from a particular layer to the adjacent one. The power efficiency of the device (conversion of the electrical power in the light flux at the given wavelength or in the given colour range) depends on (a) Joule losses caused by the overall resistance and on (b) the efficiency of conversion of charge carriers into photons. The latter depends predominantly on the charge carrier (electron-hole) balance and on the quantum efficiency of radiative recombination of the electron-hole pairs (excitons) in the device.

There has been steady effort to develop materials and OLED designs which minimize Joule losses, improve charge carrier balance and maximize the quantum efficiency. Joule losses have been reduced significantly through improved charge injecting layers and the introduction of electrically doped charge transporting layers (see e.g. WO2003/070822 or WO2005/086251 and references cited therein). Specific charge injecting and blocking layers can also improve the charge carrier balance. A further improvement in quantum efficiency has been obtained through phosphorescent emitters, which allow in theory 100 % efficiency as all excitons formed can decay radiatively. By comparison, in fluorescent emitters triplet and singled excitons are formed, but only singlet excitons can decay radiatively. Therefore, the quantum efficiency in fluorescent emitters is less than 100 %.

A number of materials used for preparing hole transport layers and/or electron/exciton blocking layers are known.

However, the OLED efficiency is still significantly below its theoretical limits and many other OLED-performance parameters like luminosity and lifetime can be also further improved.

From a practical point of view, it is advantageous that one matrix compound is suitable for various OLED designs, e.g. in phosphorescent as well as fluorescent OLEDs, and in both electrically doped hole injecting and/or hole transporting layers as well as in electrically undoped electron blocking layers.

Currently, the most important field of commercial OLED application is in displays, especially in colour AMOLED displays used in mobile phones, flat displays for personal computers, tablets, and TV sets. Another potentially important OLED application is in lighting. In polychromatic devices like polychromatic displays and polychromatic OLEDs, e.g. in state-of-the-art colour displays or in white OLEDs used for lighting purposes, the desired polychromaticity is usually achieved by employing two or more monochromatic OLEDs or by employing OLED stacks comprising more than one monochromatic emitting layers.

Generally, polychromatic displays and/or polychromatic OLEDs for lighting usually comprise at least two emitters designed for different light colours. In other words, the CIE coordinates as defined by International Commission for Illumination (see http://en.wikipedia.org/wiki/International_Commission_on_Illumination) differ in OLEDs designed for different light colours. Most usually, OLEDs or emitting layers designed separately for red (R), green (G) and blue (B) light are comprised in displays and/or white lighting devices.

In monochromatic OLEDs that are intended for a specific light chromaticity primarily by choosing an appropriate light emitting compound (emitter), optimum performance parameters (like operational voltage, efficiency and lifetime) are usually achieved through tailoring the other materials used in the OLED to fit optimally with the chosen emitter. Numerous compound classes are available for each function in the device. Even within a narrow range of physical properties, for example triplet level or frontier orbital energy levels, a wide range of materials classes are typically available. Despite exact design rules have yet to be established to be able to predict which combination of particular chemical compounds and layer thicknesses results in the best performance of the OLED device, skilled person is in principle able to find for each particular layer an optimum supporting compound that is best fitting with the chosen emitter. Consequently, in monochromatic devices optimized for maximal performance, it happens only exceptionally that one compound is applicable in two distinct layers. Polychromatic devices with reasonable performance and desired chromaticity can be designed by combining such optimized monochromatic devices appropriately.

On the other hand, the more various materials are used in a complex polychromatic device like white OLED or colour display, the more complicated is its manufacturing, and the more difficult is achieving the desired high throughput and low cost at constant high quality in mass industrial production.

Consequently, there is an unmet demand for highly versatile supporting materials for OLED like emitter hosts, hole transport materials, electron transport materials, hole blocking materials, electron blocking materials, electrical dopants and others, that could be used with all emitters chosen for a particular complex device like polychromatic display or white OLED, without substantially deteriorating performance parameters in comparison with a state-of-the-art polychromatic device of an equivalent chromaticity, wherein the comparative state-of-the-art polychromatic device comprises the same emitters and supporting materials specifically fitting with the chosen emitters.

It is therefore an object of the present invention to provide a hole transporting matrix compound with high versatility, applicable in various OLED designs, especially with both fluorescent as well as phosphorescent emitters, moreover, with emitters designed for various emission colours. In one aspect of the invention a high performance polychromatic light emitting device accessible with high throughput manufacturing processes at low cost shall be provided. In another aspect of the invention, a monochromatic OLED with high performance irrespective of the exact nature of the emitter used shall be provided.

The term "monochromatic" throughout this application shall be understood in the sense that the colour of light emitted from a monochromatic device is perceived by a human as one of the three basic colours R, G and B. Similarly, the term "polychromatic" has the meaning that the light emitted by polychromatic device has a colour that deviates from each of the three basic colours.

The object is achieved by a compound represented by formula (1)

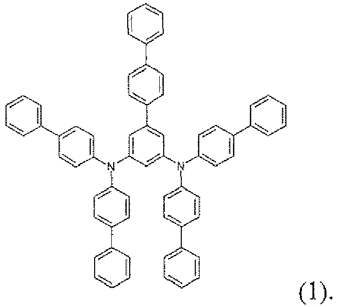

Another object of the invention is achieved by an OLED comprising between anode and cathode at least one emitter compound and at least one substantially organic layer comprising the compound represented by formula (1).

The term "substantially organic" means that majority of the overall volume of the layer consists of compounds that comprise covalent bonds carbon-carbon. Substantially organic layer may comprise organic compounds having small molecules with relative molecular weight below 1000, oligomers with relative molecular weight up to 3000, or polymers having relative molecular weight above 3000. Preferably, compound (1) forms at least half of the volume of the substantially organic layer.

The term "emitter compound", further shortened as "emitter", means that the compound has electroluminescent (EL) properties. In other words, the emitter is a compound that can emit light if placed between two electrodes in an electroluminescent device operated at appropriate voltage and current.

Preferably, the emitter is located in an emitting layer that is distinct from other layers in the device. In one of preferred embodiments, the emitter is a, preferably low-molecular, phosphorescent compound. In another preferred embodiment, the emitter is a, preferably low-molecular, fluorescent compound. More preferably, the light emitted by the phosphorescent emitter is in the blue, green, yellow or red region of the spectrum. Preferably, the fluorescent emitter emits in the violet or blue region of the spectrum.

It is preferred that the layer comprising compound (1) is located between the emitting layer and the anode. In a preferred embodiment, at least one layer containing the compound of formula (1) is electrically doped.

More preferably, the layer containing the compound of formula (1) comprises at least one electrical p-dopant and is adjacent to an electrically undoped layer comprising compound of formula (1). In a preferred embodiment, the undoped layer comprising compound (1) is adjacent to the emitting layer and serves as electron blocking layer. Also preferably, the electrically doped layer comprising compound (1) serves as the hole transporting layer. Also preferably, the electrically doped layer comprising compound (1) is adjacent to the anode.

The term "electrical doping" means generally an improvement of electrical properties, especially the electrical conductivity, in the electrically doped semiconducting material if compared with an undoped matrix material. More detailed explanation of current theory and various examples of electrical doping are available in many published patent documents, e.g. WO2014/037512.

In one yet more preferred embodiment, the electrically less doped or electrically undoped part of the layer serves as both electron-blocking and triplet exciton blocking layer.

Yet another object of the invention is achieved by polychromatic light emitting device comprising at least two OLEDs designed so that the light emitted from the OLEDs differs in its CIE coordinates, wherein all OLEDs in the device contain between cathode and anode at least one emitter and at least one substantially organic layer comprising compound represented by formula (1).

### Detailed description of the invention

Among well-known hole-transporting materials with triarylamine and benzidine structures, some alkylaryl derivatives, for example 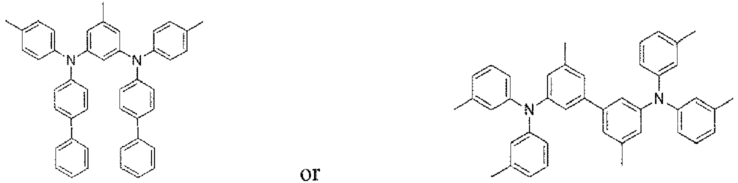 were described (see e.g. EP 687 668 or JP H03-094260).

While performing detailed investigations into performance-limiting factors, it was surprisingly found by the inventors that some derivatives with similar core structures perform unexpectedly well when used in OLEDs containing a phosphorescent emitter see PCT/EP2013/071742. Unfortunately, their performance in conventional fluorescent OLED was only moderate and especially lifetimes of experimental devices had not achieved the level provided by established hole transporting matrix materials like H-1 and H-2 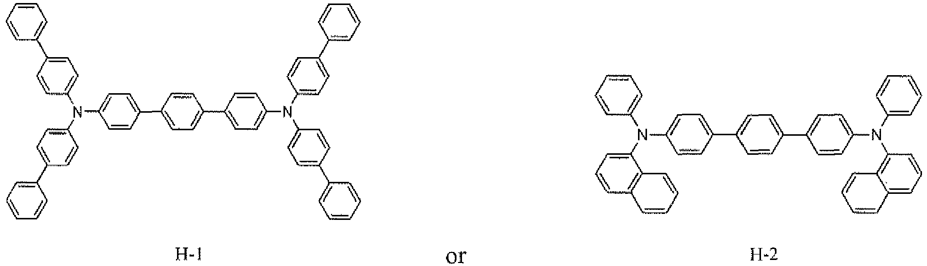 known e.g. from WO2011/134458 and US2012/223296. Compound 9 disclosed in JP H05 105647 A (Minolta Camera KK) 27 April 1993 is considered to be the closest prior art. Therefore, further improvements in performance are still required. Through further research into high performance OLEDs with both fluorescent as well as phosphorescent emitters, the inventors finally arrived at compound (1). It was surprisingly found that whereas the performance of the compound (1) in phosphorescent devices is comparable with best compounds of PCT/EP2013/071742 in terms of operating voltage and only slightly worse in terms of device efficiency, compound (1) outperforms compounds of PCT/EP2013/071742 if used as hole transporting and electron blocking layer in devices containing fluorescent emitters.

Emitting layer, electron transporting layer, hole blocking layer, electrodes

Other parts of inventive light emitting devices than the inventive hole transporting and/or electron blocking layer can be prepared in various designs and from various materials described in the scientific and patent literature, e.g. in patent documents cited throughout this application.

In the examples, following supporting materials were used: 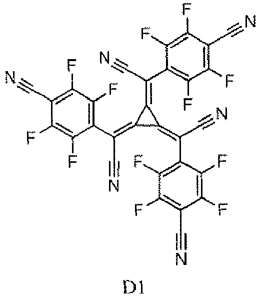 as a p-dopant (US 2010/102709), 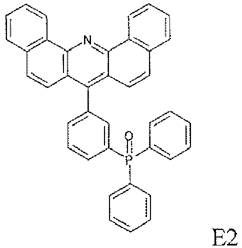 as electron-transporting matrix (WO 2013/079217), 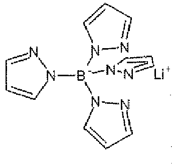 D3 as n-dopant (WO 2013/079676), 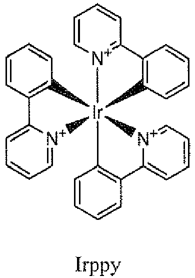 as a well-known triplet green emitter, 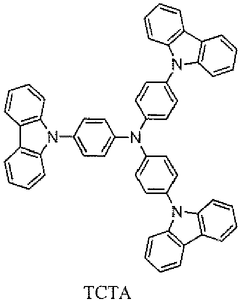 as a well-known electron blocking matrix.

### Description of drawings

Figure 1: Schematic drawing of experimental bottom emitting phosphorescent OLED
Figure 2:
   a) Top view of deposition of layer 1 (p-doped inventive material (stripes), p-doped reference (dots), left;
   b) Top view of layer 2 after rotation of substrate by 90°, with the inventive material in the top row (fields A, C) and reference material in the bottom row (fields B, D).

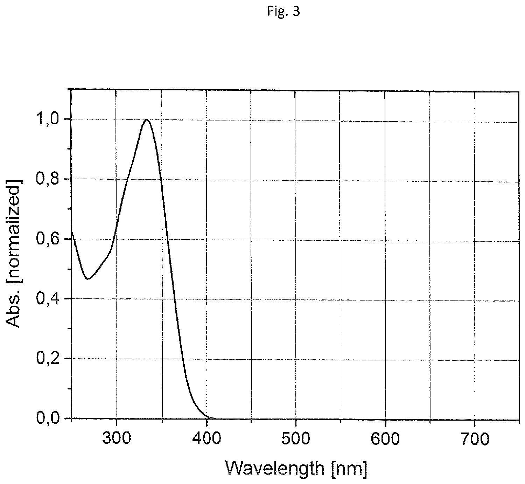

Figure 3: Spectrum of compound (1) in the ultraviolet-visible (UV-vis) range.

### Examples

### 1. Synthesis of inventive material

### General procedure for 3,5-dibromophenylenes

1,3,5-Tribromobenzene, the boronic acid and Pd(PPh₃)₄ were dissolved in a mixture of toluene and ethanol. A degassed 2M aqueous Na₂CO₃ solution was added. The mixture was refluxed for 18 hours. After cooling to room temperature the organic phase was separated from the aqueous one. The aqueous phase was extracted with toluene three times. The combined organic phases were evaporated to dryness and the residue was filtered over a pad of silica gel using dichloromethane (DCM) as eluent. After evaporating the solvents the crude product was purified by column chromatography on silica gel using hexane : DCM mixtures as an eluent. In thin layer chromatography (TLC), the upper main spot was identified as the desired product and the one below as the 3,5-disubstituted bromobenzene side product.

### 3,5-dihromo-1,1':4',1"-terphenyl

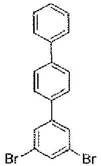

1,3,5-tribromobenzene: 10.00 g (1.2 eq, 31.77 mmol)
4-biphenylboronic acid: 5.24 g (1.0 eq, 26.47 mmol)
Pd(PPh₃)₄: 612 mg (2 mol%, 0.53 mmol)
toluene: 160 mL
ethanol: 52 mL
2MNa₂CO₃:26mL
Yield: 4.95 g (48%)
GC-MS: m/z = 386 / 388 / 390

### General procedure for secondary amines

Under an inert atmosphere the bromoaryl component, palladium(II)acetate, caesium carbonate and 2,2'-bis(diphenylphosphino)-1,1'-binaphthyl (BINAP) were combined in a flask and dissolved in 1,4-dioxane. The primary arylamine component was added, followed by heating up the mixture to reflux and stirring for 18-48 hours. According to TLC the reaction was complete. The mixture was cooled to room temperature and filtered through a pad of silica gel. After washing with DCM and evaporation of the solvent the crude product was purified by column chromatography (SiO₂, hexane:DCM mixtures). The combined fractions were evaporated to dryness and the resulting solid was recrystalized from hexane to yield the desired product.

### di([1,1'-biphenyl]-4-yl)amine

| | |
|---|---|
| 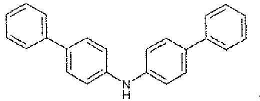 | 20.00 g (1.0 eq, 85.80 mmol) |
| 4-bromobiphenyl | |
| 1,1'-biphenyl-4-amine | 15.25 g (1.05 eq, 90.10 mmol) |
| palladium(II) acetate | 578 mg (3.0 mol.%, 2.57 mmol) |
| BINAP | 2.40 g (4.5 mol.%, 3.86 mmol) |
| caesium carbonate | 39.10 g (1.4 eq, 120.12 mmol) |
| 1,4-dioxane | 200 mL |
| reflux time | 42 h |

### General procedure for tertiary amines of the 3,5-diaminophenylene class

Under an inert atmosphere, the secondary amine, the dibromo compound, bis(dibenzylidenaceton)palladium, tri-*tert*-butylphosphine and potassium-*tert*-butoxide were combined in a flask and solved in toluene. The mixture was stirred at 80°C for two hours and then cooled to room temperature. TLC indicated complete consumption of the starting materials. The mixture was filtered through a pad of silica gel, washed with DCM and the filtrate evaporated to dryness. The crude solid product was stirred in hot toluene. After cooling to room temperature, the mixture was filtered to yield the product. Finally, the product purified by gradient sublimation under high vacuum (10⁻⁶ mbar) condition.

### N3,N3,N5,N5-tetra([1,1'-biphenyl]-4-yl)-[1,1':4',1"-terphenyl]-3,5'-diamine (1)

| | |
|---|---|
| di([1,1'-biphenyl]-4-yl)amine | 5.40 g (2.1 eq, 16.80 mmol) |
| 4,4"-dibromo-1,1':4',1"-terphenyl | 3.10 g (1.0 eq, 7.99 mmol) |
| bis(dibenzylidene-acetone) palladium | 87 mg (2.0 mol.%, 0.31 mmol) |
| tri-*tert*-butylphosphine | 46 mg (3.0 mol.%, 0.46 mmol) |
| potassium-*tert*-butoxide | 2.54 g (3.0 eq, 46.4 mmol) |
| toluene | 225 mL |

Yield before sublimation: 5.48 g (78 %)
¹H NMR (CD₂Cl₂): δ = 7.61-7.58 (m, 4H), 7.58-7.52 (m, 18H), 7.46-7.37 (m, 10H), 7.36-7.30 (m, 4H), 7.30-7.26 (m, 8H), 7.13 (d, J = 2.0 Hz, 2H), 6.94 (t, J = 2.0 Hz, 1H) ppm.
¹³C NMR (CD₂Cl₂): δ = 149.51, 147.23, 143.30, 141.04, 141.01, 140.85, 140.15, 136.18, 129.37, 129.35, 128.37, 127.97, 127.90, 127.86, 127.47, 127.46, 127.16, 125.03, 119.51, 117.62 ppm.

Differential scanning calorimetry (DSC):
m.p. 292 °C (peak temperature at heating rate 10 K/min)
T_{g} 133 °C (onset temperature at heating rate 10 K/min)

### 2. OLED preparation and testing

Performance testing of the new material was carried out as explained in detail for bottom emitting phosphorescent organic light emitting diode of Example 1. The diodes were processed in vacuum via vapor thermal deposition of organic materials (active layers) and metals (electrodes). Shadow mask techniques were used to structure the devices (active matrix, electrodes).

### Example 1: bottom emitting green phosphorescent OLED

Four OLEDs are prepared on one substrate with an active area of 6.70 mm² each. 16 identical indium tin oxide (ITO) substrates with 90 nm thick ITO layer serving in prepared OLEDs as an anode were processed at once in a 4x4 array placed on a table which is pivotable around its vertical axe. Using shutters, each of these 16 substrates can be covered by different set of organic layers.

The ITO substrates were cleaned and put into a vapor thermal deposition unit in the 4x4 array. A reference p-doped layer (e.g. H-1 doped with D1; molar ratio (97:3) was deposited on half of these substrates for a final film thickness of 60 nm. On the other half of the plate, the studied inventive material was codeposited with the same p-dopant at the same 97:3 molar ratio and thickness. After a rotation of the plate by 90°, the second (electron blocking) layer is deposited on top of the first layer. Here, half the plate is covered with 20 nm of the reference compound (e.g., TCTA) and the other half with the same inventive material as used in the first layer (see figure 1).

The reference devices (figure 1, field D) were thus always processed together with the devices comprising the inventive material. This approach allows assessing performance of new material in comparison with the reference independent from possible day-to-day variations of deposition rates, vacuum quality or other tool performance parameters. As each field contains 16 identically prepared OLEDs and the performance parameters were estimated for each of these 16 OLEDs, statistical evaluation of the obtained experimental results unequivocally showed the statistical significance of the observed average values reported in the Table 1.

The subsequent phosphorescent green emission layer (Merck_TMM004:Irrpy at weight ratio 9:1) was deposited with a thickness of 20 nm, followed by 20 nm Merck_TMM004 as a hole blocking layer and 25 nm E-2 layer doped with D3 (matrix to dopant weight ratio 4:1). The cathode was prepared by vacuum deposition of 100 nm aluminum layer.

### Example 2: bottom emitting blue fluorescent OLED

Bottom emitting blue fluorescent OLEDs were prepared on ITO substrates and tested analogously, with differences in used materials and thicknesses of deposited layers as follows. 10 nm thick hole injection layer consisting of a chosen hole transporting matrix and D1 in weight ratio 92:8 was deposited on the cleaned ITO surface, followed by 120 nm thick neat layer of the chosen electron blocking matrix. Then, Sun Fine Chem (SFC, Korea) host ABH113 and blue emitter NUBD370 were codeposited in the weight ratio 97:3 as a 20 nm thick emitting layer, followed by 36 nm thick electron transporting layer consisting of 60 weight % E2 and 40 weight % lithium 8-hygroxyquinoline salt (LiQ). The 100 nm aluminium cathode was deposited on top of the electron transporting layer.

### 3. Technical effect of the invention

Table 1 shows the experimental results obtained by the procedure described in detail in the Example 1 below. The green OLED generally represents all monochromatic phosphorescent OLEDs. In Example 1, the hole transporting layer was doped with a p-dopant, what is symbolized with the p- symbol in the substrate/HTL/EBL column. In the table, to the compounds showing lower voltage than reference, negative values were assigned in the voltage column. Oppositely, a positive value in the voltage column shows unfavourable, higher average voltage observed at the set of devices comprising inventive compound in comparison with the average voltage measured on the set of reference devices prepared under the same conditions. In the efficiency column, the average efficiency of devices comprising an inventive compound higher than the average efficiency of comparative devices is positive, whereas unfavourable lower efficiency in comparison with reference has negative sign. The column assigned in the table as Q - voltage shows the arithmetic difference between the value in the efficiency column and the value in the voltage column. The resulting value was used as a benchmark for assessing the overall performance. Its positive value in at least one from the three rows shows that at least in one application - if the compound was used as an EBL, as an HTL, or in both layers - shows that in this particular case, the percentage voltage improvement has overweighed the percentage efficiency decrease or, oppositely, that the percentage efficiency improvement overweighed the undesired voltage increase, or that there was an improvement in both properties.

**Table 1**

| **Compound tested** | **phosphorescent green OLED** | | | |
|---|---|---|---|---|
| | substrate/HTL/EBL | voltage change [%] | Q eff change [%] | Q - voltage [%] |
| H-1 | ITO / p-H-1 /H-1 | -8 | -38 | -30 |
| H-2 | ITO / p-H-1 / H-2 | -8 | -49 | -41 |
| | ITO / p-H-2 /H-2 | -8 | -50 | -42 |
| TCTA | ITO / p-H-1 / TCTA | 0 | | reference |
| | ITO / p-TCTA / TCTA | +38 | +5 | -33 |
| (1) | ITO / p-H-1 / (1) | -10 | -12 | -2 |
| | ITO / p-(1)/ TCTA | +2 | -1 | -3 |
| | ITO / p-(1)/ (1) | -6 | -14 | -8 |

It is clearly seen that in phosphorescent OLEDs, the state-of-the-art matrices for a doped hole transporting layer and for undoped electron blocking layer are no way interchangeable or applicable in both layers without significant deterioration of the overall performance score shown by highly negative value in the last column.

Oppositely, (1) can be used as a matrix equally well in p-doped HTL, undoped EBL, as well as in both layers, without a significant deterioration of the overall performance score.

Additionally, it has been found that inventive compound is applicable without significant performance deterioration also when used as hole transporting and/or electron blocking matrix in blue fluorescent OLED of example 2, representing fluorescent OLEDs generally. As the lifetime is often insufficient in blue OLEDs, a comparison of the LT-97 (mean time in hours necessary for 3 % change of the initial lumince in experimental devices operated at current density 15 mA/cm² at normal temperature) is included. The comparison of a device built using compound (1) with the device comprising the state-of-the-art HTL and EBL matrices is shown in the Table 2.

**Table 2**

| **Compound tested** | **fluorescent blue OLED** | | | | |
|---|---|---|---|---|---|
| | substrate/HTL/EBL | voltage change [%] | Q eff change [%] | Q - voltage [%] | LT-97 [%] |
| H-2 | ITO / p- H-2/ H-2 | 0 | | reference | 0 |
| (1) | ITO p-(1)/ (1) | -2 | +8 | +10 | -14 |

In fluorescent OLEDs, matrices like H-1 or H-2 are generally applicable in both HTL as well as EBL matrices. It is clearly seen that also in blue OLEDs, compound (1) can replace known matrix materials without remarkable performance deterioration.

The features disclosed in the foregoing description and in the claims may, both separately and in any combination, be material for realizing the invention in diverse forms thereof.

## Claims

1. Polychromatic light emitting device comprising at least two OLEDs designed so that the light emitted from the OLEDs differs in its CIE coordinates, wherein all OLEDs in the device comprise between cathode and anode at least one emitter and at least one substantially organic layer comprising compound represented by formula (1)

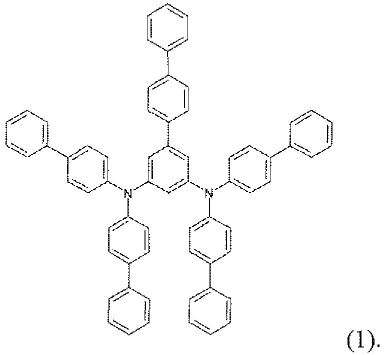

2. OLED comprising between anode and cathode at least one emitter compound and at least one substantially organic layer comprising compound represented by formula (1)

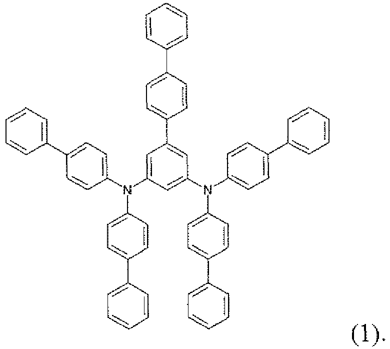

3. OLED according to claim 2, wherein the emitter is a, preferably low-molecular, fluorescent or phosphorescent compound.

4. OLED according to any of claims 2-3, wherein the emitter is located in an emitting layer that is distinct from other layers in the device.

5. OLED according to claim 4, wherein the layer comprising compound (1) is located between emitting layer and anode.

6. OLED according to any of claims 2-5, wherein at least one layer containing the compound of formula (1) is electrically doped with at least one p-dopant.

7. OLED according to any of claims 4-6 wherein at least one layer containing the compound of formula (1) is adjacent to the emitting layer.

8. OLED according to claim 6 or 7, wherein the layer containing compound of formula (1) and at least one p-dopant is adjacent to another electrically undoped layer comprising compound of formula (1).

9. OLED according to any of claims 2-8, wherein at least one layer containing the compound of formula (1) is adjacent to the anode.

10. Compound represented by formula (1)

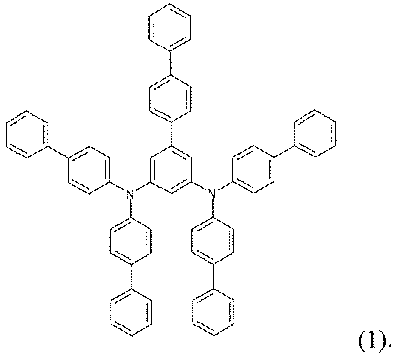

## Patentansprüche

1. Vorrichtung zum Emittieren von polychromatischem Licht, umfassend zumindest zwei OLEDs, die derart gestaltet sind, dass Licht, welches von den OLEDs emittiert wird, sich in seinen CIE Koordinaten unterscheidet, wobei alle OLEDs in der Vorrichtung zwischen einer Kathode und einer Anode zumindest einen Emitter und zumindest eine im Wesentlichen organische Schicht, die eine Verbindung, dargestellt durch die Formel (I), umfasst, umfassen

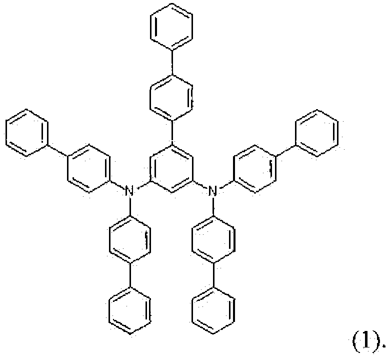

2. OLED, die zwischen einer Anode und einer Kathode zumindest eine Emitterverbindung und zumindest eine im Wesentlichen organische Schicht, die eine Verbindung, die dargestellt wird durch die Formel (1), umfasst, umfasst

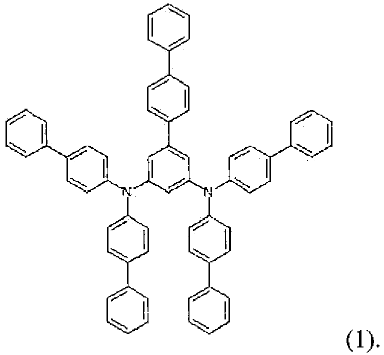

3. OLED nach Anspruch 2, wobei der Emitter eine, vorzugsweise niedermolekulare, fluoreszierende oder phosphoreszierende Verbindung ist.

4. OLED nach einem der Ansprüche 2 oder 3, wobei der Emitter in einer emittierenden Schicht angeordnet ist, die abgegrenzt ist von den anderen Schichten der Vorrichtung.

5. OLED nach Anspruch 4, wobei die Schicht, die die Verbindung (1) umfasst, zwischen der emittierenden Schicht und der Anode angeordnet ist.

6. OLED nach einem der Ansprüche 2 - 5, wobei zumindest eine Schicht, die die Verbindung der Formel (1) enthält, elektrisch mit zumindest einem p-Dotanden dotiert ist.

7. OLED nach einem der Ansprüche 4 - 6, wobei zumindest eine Schicht, die die Verbindung der Formel (1) enthält, benachbart zu der emittierenden Schicht ist.

8. OLED nach Anspruch 6 oder 7, wobei die Schicht, die die Verbindung der Formel (1) und zumindest einen p-Dotand enthält, benachbart ist zu einer anderen elektrisch undotierten Schicht, die die Verbindung nach Formel (1) umfasst.

9. OLED nach einem der Ansprüche 2 - 8, wobei zumindest eine Schicht, die die Verbindung der Formel (1) enthält, benachbart zu der Anode ist.

10. Verbindung, die dargestellt ist durch die Formel (1)

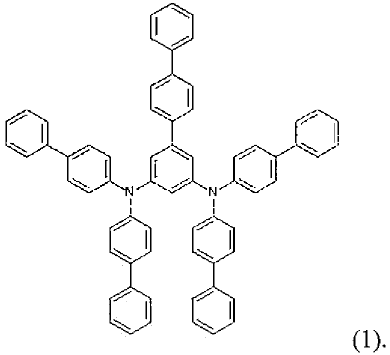

## Revendications

1. Dispositif électroluminescent polychromatique comprenant au moins deux OLED conçues de sorte que les lumières émises des OLED aient des coordonnées CIE différentes, dans lequel toutes les OLED dans le dispositif comprennent, entre une cathode et une anode, au moins un émetteur et au moins une couche sensiblement organique comprenant un composé représenté par la formule (1) :

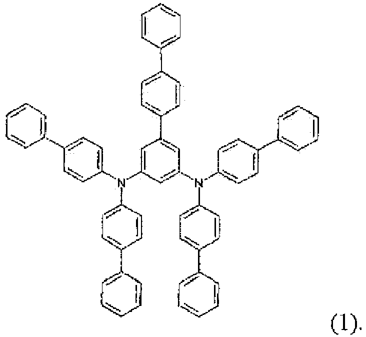

2. OLED comprenant, entre une anode et une cathode, au moins un composé émetteur et au moins une couche sensiblement organique comprenant un composé représenté par la formule (1) :

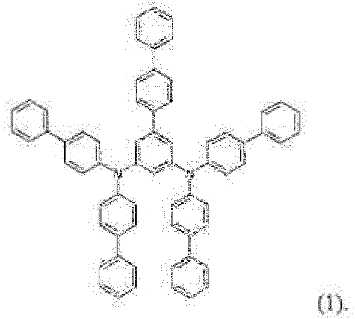

3. OLED selon la revendication 2, dans laquelle l'émetteur est un composé fluorescent ou phosphorescent, de préférence de faible poids moléculaire.

4. OLED selon l'une quelconque des revendications 2 et 3, dans laquelle l'émetteur est situé dans une couche émettrice qui est distincte d'autres couches dans le dispositif.

5. OLED selon la revendication 4, dans laquelle la couche comprenant le composé (1) est située entre une couche émettrice et une anode.

6. OLED selon l'une quelconque des revendications 2 à 5, dans laquelle au moins une couche contenant le composé de la formule (1) est électriquement dopée avec au moins un dopant p.

7. OLED selon l'une quelconque des revendications 4 à 6, dans laquelle au moins une couche contenant le composé de la formule (1) est adjacente à la couche émettrice.

8. OLED selon la revendication 6 ou 7, dans laquelle la couche contenant le composé de la formule (1) et au moins un dopant p est adjacente à une autre couche électriquement non dopée comprenant le composé de la formule (1).

9. OLED selon l'une quelconque des revendications 2 à 8, dans laquelle au moins une couche contenant le composé de la formule (1) est adjacente à l'anode.

10. Composé représenté par la formule (1) :
